# EUROPÄISCHE PATENTSCHRIFT

(11) **EP 4 132 365 B1**
(45) Veröffentlichungstag und Bekanntmachung des Hinweises auf die Patenterteilung: **08.01.2025**
(21) Anmeldenummer: 21714815.4
(22) Anmeldetag: 18.03.2021
(51) Int. Cl.: A61B 8/08, A61B 8/00, G06F 3/01, G06T 19/00

(54) **ULTRASCHALL-ERWEITERTE REALITÄT-PERIPHER ENDOVASKULÄR INTERVENTION-NAVIGATIONSANORDNUNG**
ASSEMBLY FOR NAVIGATING AN ULTRASOUND AUGMENTED REALITY PERIPHERAL ENDOVASCULAR INTERVENTION
ENSEMBLE POUR NAVIGUER DANS UNE INTERVENTION ENDOVASCULAIRE PÉRIPHÉRIQUE PAR ULTRASONS À RÉALITÉ AUGMENTÉE

(30) Priorität: 06.04.2020 DE 102020109593
(43) Veröffentlichungstag der Anmeldung: 15.02.2023
(73) Patentinhaber: Universität zu Lübeck, 23562 Lübeck (DE)
(72) Erfinder: PREUß, Mark, 24159 Kiel (DE); KLEEMANN, Markus, 23552 Lübeck (DE); ERNST, Floris, 23627 Groß Grönau (DE)
(74) Vertreter: Puschmann Borchert Kaiser Klettner Patentanwälte Partnerschaft mbB
(86) Internationale Anmeldenummer: PCT/DE2021/100275
(87) Internationale Veröffentlichungsnummer: WO 2021/204325

(56) Entgegenhaltungen:
- WO-A1-2018/211235
- US-A1- 2006 176 242
- KLEEMANN M ET AL: "Robotik: automatisierte Verfahren in der Gefäßmedizin", GEFAESSCHIRURGIE, SPRINGER, HEIDELBERG, DE, vol. 24, no. 7, 21 October 2019 (2019-10-21), pages 557 - 563, XP036916001, ISSN: 0948-7034, [retrieved on 20191021], DOI: 10.1007/S00772-019-00581-8

## Beschreibung

Die Erfindung betrifft eine Ultraschall-Erweiterte Realität-Peripher Endovaskulär Intervention-Navigationsanordnung.

Die Etablierung endovaskulärer Techniken hat die Welt der konventionellen offenen Gefäßchirurgie grundlegend verändert und eine neue Ära der Behandlung gefäßchirurgischer Patienten eingeleitet. Dank endovaskulärer Techniken können Patienten heutzutage minimal invasiv ohne großes Zugangstrauma behandelt werden. Dies erlaubt auch die Behandlung von Patienten, die einer konventionell offen chirurgischen Behandlung aus gesundheitlichen Gründen nicht mehr zugänglich sind. Des Weiteren haben endovaskulär behandelte Patienten weniger postoperative Komplikationen und somit eine kürzere Krankenhausverweildauer.

In den letzten Jahrzehnten hat sich die Anwendung endovaskulärer Techniken aus den zuvor genannten Gründen rasant entwickelt, nicht zuletzt, weil auch die Industrie hier einen neuen Markt entdeckt hat. Endovaskuläre Eingriffe gehören heute zum Standardrepertoire eines jeden Gefäßchirurgen und verdrängen mehr und mehr die konventionellen offenen chirurgischen Eingriffe. Für einen Großteil der peripheren Gefäßpathologien wird heutzutage ein endovaskulärer Eingriff empfohlen. Schätzungen zufolge werden im Jahr 2026 75 bis 95 % der gefäßchirurgischen Eingriffe endovaskulär durchgeführt werden.

**Problematisch im Stand der Technik** ist im Wesentlichen, dass für die Durchführung von endovaskulären Interventionen die Anwendung von Röntgenstrahlen notwendig ist. Zwar haben der technische Fortschritt sowie die Befolgung von Strahlenschutzmaßnahmen bereits zu einer deutlichen Strahlenreduktion geführt, jedoch kommt es bedingt durch die immer komplexeren Prozeduren, die eine lange Durchleuchtungszeit benötigen, zu einer zum Teil erheblichen Strahlenbelastung für den Patienten und den Operateur. Zum Zwecke der Qualitätssicherung werden daher durch gesetzliche Regelungen Dosisflächenprodukte je Eingriff empfohlen, deren Überschreitung dokumentiert werden muss und in die Qualitätssicherung eingeht.

Nachteilig bei der Anwendung von Röntgenstrahlen im Rahmen endovaskulärer Interventionen ist des Weiteren die Gefahr einer Nierenschädigung durch Applikation von Kontrastmittel. Als Genese wird ein Kontrastmittel induzierter hypoxischer Nierenschaden angenommen. Die Inzidenz akuter Nierenschäden bei peripheren Interventionen beträgt bis zu 10 %. Die Leitlinie der Deutschen Gesellschaft für Nephrologie empfiehlt daher eine Risikoabschätzung, umfassend insbesondere ein Screening auf eine präexisitierende Einschränkung der Nierenfunktion, bei allen Patienten vor Untersuchungen mit intravaskulärer Gabe von jodhaltigem Kontrastmittel. Als protektive Maßnahmen werden eine intravenöse Volumenexpansion mit isotonischer Kochsalzlösung oder Natriumbikarbonatlösung sowie die Gabe von N-Acetylcystein oral zusammen mit intravenöser isotonischer Kristalloidlösung empfohlen. Die beste Prävention ist jedoch die geringste mögliche Kontrastmitteldosis zu applizieren beziehungsweise alternative Methoden der Bildgebung ohne Kontrastmittel in Betracht zu ziehen.

Ein weiteres Problem ist zudem, dass unter Röntgendurchleuchtung nur eine zweidimensionale Navigation in einer dreidimensionalen anatomischen Struktur möglich ist. Dies macht die Navigation, insbesondere für komplexe Eingriffe, schwierig und führt zu längeren Durchleuchtungszeiten und somit zu einer vermehrten Gabe von Kontrastmittel.

Aus dem **Stand der Technik** sind unterschiedliche Ansätze zur Verminderung der Strahlenexposition bekannt. Ein Ansatz besteht im vermehrten Einsatz der Sonographie.

In der Druckschrift Abbas A., Hansrani V., Sedgwick N. et al. (2014) "3D contrast enhanced ultrasound for detecting endoleak foolowing endovascular aneuyrism repair (EVAR)", European Journal of Vascular and Endovascular Surgery 47: 487-492, doi: 10.1016/j.ejvs.2014.02.002 wird beschrieben, dass neben der Darstellung peripherer Gefäße heutzutage die Möglichkeit besteht mit kontrastverstärktem Ultraschall und dreidimensionaler Technologie sicher Endoleaks nach Implantation von Aortenstentprothesen zu detektieren. Vor allem in der Diagnostik und Nachsorge von Gefäßpathologien ist die Duplexsonographie daher mittlerweile zum Diagnostikum der ersten Wahl avanciert. Die Qualität von Ultraschalluntersuchungen ist jedoch sehr spezifisch in Bezug auf Untersucher und Patient. Zudem ist nicht jede Körperregion von außen einer Ultraschalluntersuchung zugänglich. Eine praktische Nutzung für die Navigation endovaskulärer Eingriffe ist daher bis dato nicht umgesetzt.

Eine Weiterentwicklung ist die intravaskuläre Sonographie. Im Vergleich zur konventionellen Angiographie liefert diese ein detailliertes Bild der Gefäßwand. Des Weiteren ist eine dreidimensionale Rekonstruktion von Gefäßpathologien möglich. Die intravaskuläre Sonographie konnte daher eine Überlegenheit bei der Differenzierung von Stenosen und Re-Stenosen gegenüber der konventionellen Angiographie aufzeigen.

Eine ähnliche Technik ist die optische Kohärenztomographie. Hierbei wird das Gefäß nicht mit Ultraschall sondern mit Licht erfasst. Das Ergebnis ist ein dem Ergebnis der intravaskulären Sonographie entsprechendes Bild, jedoch mit deutlich besserer Auflösung. Sowohl die Anwendung der intravaskulären Sonographie als auch der optischen Kohärenztomographie sind bei peripheren Interventionen beispielsweise in der Druckschrift Secco G., Garatoni C., Parisi R. et. al. (2015), "Optical Coherence Tomography Guidance during Peripheral Vascular Intervention", Cardiovascular and Interventional Radiology 38: 768-772, doi: 10.1007/s00270-014-0868-3 oder in der Druckschrift Karnabatidis D., Katsanos K., Paraskevopoulos I. et. al. (2011), "Frequency-domain intravascular optical coherence tomography of the femoropopliteal artery", Cardiovascular and Interventional Radiology 34: 1172-1181, doi: 10.1007/s00270-010-0092-8 beschrieben.

Es handelt sich bei beiden Techniken bis dato jedoch nur um eine additive Anwendung im Rahmen einer konventionellen Intervention. Für die Navigation der endovaskulären Intervention wird nach wie vor Röntgenstrahlung benötigt. Nichtsdestotrotz liefern beide Techniken differenzierte Informationen über die innere Gefäßbeschaffenheit.

Der medizinische Nutzen für den Patienten steht demnach immer noch der Strahlenexposition mit all ihren Risiken für den Organismus gegenüber. Ob es zu einem gesundheitlichen Schaden kommt hängt von der absorbierten Strahlenmenge, der Strahlenart und davon ab, welches Organ oder Gewebe des Körpers hauptsächlich betroffen ist. Bei den Strahlenschäden wird grundsätzlich zwischen deterministischen und stochastischen Schäden unterschieden.

Deterministische Schäden sind in der Regel die Folge einer massiven Abtötung von Zellen in einem Organ- oder Gewebesystem durch Apoptose oder Nekrose. Werden zu viele Zellen in einem Gewebe oder Organ abgetötet, kommt es zu Funktionseinbußen in dem betroffenen Organ. Deterministische Strahlenschäden werden durch hohe Dosen an ionisierender Strahlung hervorgerufen und treten bei Überschreitung eines Schwellenwertes auf.

Stochastische Strahlenschäden entstehen, wenn es in Folge einer Strahlenexposition zu einer unzureichenden oder fehlerhaften Reparatur der DNA kommt und die genetische Information einer Zelle verändert wird. Beim natürlichen Prozess der Zellteilung vermehren sich dann die veränderten / mutierten Zellen. Dieser Vorgang kann bei Körperzellen Jahre nach der Strahlenexposition zur Entstehung von Krebs führen. Wird die Erbinformation in den Keimzellen, die in den Hoden beziehungsweise Eierstöcken produziert werden, verändert, kann dies in den darauffolgenden Generationen zu Erbschäden führen. Das Auftreten von strahlenbedingten Erbschäden wurde allerdings bisher beim Menschen nicht beobachtet, wohl aber in Studien an Tieren. Stochastische Strahlenschäden können in Körperzellen und in Keimzellen auch bei niedrigen Dosen ionisierender Strahlung auftreten. Das bedeutet, dass sowohl bei niedrigen als auch bei hohen Dosen ein stochastischer Schaden eintreten kann, aber nicht muss. Die Wahrscheinlichkeit, dass ein derartiger Strahlenschaden eintritt, wird mit zunehmender Strahlenexposition größer. Zwischen der Einwirkung der Strahlung und der Erkrankung kann eine lange Zeit vergehen.

Die Entwicklung neuer röntgenstrahlungsfreier Navigationssysteme zur Durchführung vaskulärer Interventionen ist somit von hoher klinischer Relevanz.

Aus dem BMBF geförderten Nav-EVAR-Projekt (BMBF FKZ: 13GW0228A) und den hieraus resultierenden Druckschriften Garcia-Vasquez V., Haxthausen v. F. et. al. (2018), "Navigation and Visualisation with HoloLens in endovascular Aortic repair", Innovative Surgical Sciences 3 (3): 167-177, doi: 10.1515/iss-2018-2001 und Horn M., Nolde J. et. al. (2015), "Ein Prototyp für die navigierte Implantation von Aortenstentprothesen zur Reduzierung der Kontrastmittel- und Strahlenbelastung: Das Nav-CARS-EVAR-Konzept", Zentralblatt für Chirurgie 140: 493-499, doi: 10.1055/s-0035-1546261 ist ein Navigationssystem bekannt, durch das durch den Einsatz von Erweiterter Realität endovaskuläre Interventionen mit vermindertem Einsatz von Kontrastmittel und Strahlungsdosis durchgeführt werden sollen. Mithilfe einer HoloLens / Erweiterte Realität-Brille soll der zu behandelnde Gefäßabschnitt inklusive des Zugangsbereiches auf den Patienten projiziert werden und so eine endovaskuläre Navigation der Interventionselemente ermöglicht werden. Der Datensatz für die computergenerierte Rekonstruktion des Gefäßabschnittes soll hierbei einer standardmäßig präinterventionell durchgeführten CT-Angiographie entnommen werden. Die Position des für die Intervention genutzten Katheters soll über ein elektromagnetisches Verfolgungssystem in Echtzeit über die Erweiterte Realität-Brille in einer aus den ermittelten Daten gewonnenen virtuellen Angiographie abgebildet werden.

Die Druckschrift WO 2018/211235 A1 offenbart ein Visualisierungssystem für das Nadeln. Das Visualisierungssystem ermöglicht es dem Benutzer, der ein Chirurg sein kann, die Position der Nadel und die Position der Organe innerhalb des Volumens, an dem der Eingriff durchgeführt wird, zu erkennen.

In der Druckschrift US 2019 / 0 223 958 A1 ist ein System und Verfahren zur verbesserten Navigation von medizinischen Geräten offenbart, wobei hier ein Beispielsystem einen Prozessor enthalten kann, der so konfiguriert ist, dass er eine Platzierung eines medizinischen 2D-Bildes in einem virtuellen 3D-Raum bestimmt, eine Platzierung eines virtuellen medizinischen Geräts in dem 3D-Raum bestimmt, einen Schnittpunkt basierend auf der Platzierung des medizinischen 2D-Bildes und der Platzierung des virtuellen medizinischen Geräts bestimmt und/oder einen dynamischen Projektionspunkt für das virtuelle medizinische Gerät zumindest teilweise basierend auf dem bestimmten Schnittpunkt bestimmt. Der Prozessor kann eine Anzeige veranlassen, ein Rendering des medizinischen 2D-Bildes und eine Projektion des virtuellen medizinischen Geräts auf das medizinische 2D-Bild aus einer Perspektive des dynamischen Projektionspunkts anzuzeigen. Die Anzeige kann eine Anzeige umfassen, die kommunikativ mit einem bildgebenden medizinischen Gerät gekoppelt ist. Der Sichtbereich des kann parallel zu einem 2D-Bereich sein, der mit dem medizinischen 2D-Bild verbunden ist.

Weiter ist aus der Druckschrift DE 10 2014 226 240 A1 ein System und eine Verfahren zur roboterunterstützten medizinischen Behandlung eines Patienten bekannt, wobei das System einen Manipulator, ein medizinisches Visualisierungsgerät, welches am Manipulator angebracht ist, um vom Manipulator bewegt zu werden, und ein medizinisches Instrument, welches mit zumindest einem Marker versehen ist, um die Lage des medizinischen Instruments erfassen zu können, umfasst, und wobei der Manipulator dabei das Visualisierungsgerät derart bewegen soll, dass es abhängig von der Lage bzw. Position des medizinischen Instruments orientiert wird.

Ferner ist aus der Druckschrift DE 10 2011 005 917 A1 ein medizinischer Arbeitsplatz offenbart, der ein medizinisches Instrument, welches vorgesehen ist für eine Behandlung eines Lebewesens zumindest teilweise in dessen Inneraum eingeführt zu werden, eine bildgebende Vorrichtung, die eingerichtet ist, während der Behandlung Bilddatensatze vom Inneren des Lebewesens zu erstellen, und einen Roboter aufweist, wobei der Roboter einen mehrere, hintereinander angeordnete Glieder aufweisenden Roboterarm umfasst, an dem die bildgebende Vorrichtung oder das medizinische Instrument anordenbar ist, und eine zum Bewegen des Roboterarms vorgesehene Steuervorrichtung, die eingerichtet ist, den Roboterarm derart zu bewegen, sodass die am Roboterarm befestigten bildgebenden Vorrichtung einer Bewegung des medizinischen Instruments folgt oder das am Roboterarm befestigte medizinische Instrument einer Bewegung der bildgebenden Vorrichtung folgt.

Die **Probleme im Stand** der **Technik** sind wie zuvor beschrieben im Wesentlichen, dass für die Navigation in peripheren endovaskulären Interventionen bis dato ein Verzicht auf Röntgenstrahlen und damit einhergehend die Gabe von Kontrastmittel nicht möglich ist. Erste Ansätze zur Verminderung der Strahlungsexposition durch alternative Verfahren sind in der Literatur beschrieben, ein vollkommener Verzicht ist jedoch bisher nicht möglich.

Der vorliegenden Erfindung liegt die **Aufgabe** zugrunde, eine röntgenstrahlungsfreie und kontrastmittellose Navigationsanordnung zur Durchführung einer peripheren endovaskulären Intervention bereitzustellen.

**Gelöst** wird diese Aufgabe mit einer Ultraschall-Erweiterte Realität-Peripher Endovaskulär Intervention-Navigationsanordnung gemäß Hauptanspruch.

Die Ultraschall-Erweiterte Realität-Peripher Endovaskulär Intervention-Navigationsanordnung, umfasst eine Erweiterte Realität-Vorrichtung gemäss Anspruch 1.

Der Rechner kann zudem einen Zugriff auf das Materiallogistiksystem aufweisen, wobei über die Systemdatenbank des Materiallogistiksystems anhand der Ultraschallergebnisse Materialvorschläge für das Interventionsmaterial ausgegeben werden und verbrauchtes Material markiert wird.

Das Ultraschall-Erweiterte Realität-Peripher Endovaskulär Intervention-Navigationsverfahren, welches nicht Teil der vorliegenden Erfindung ist, weist die nachfolgenden Schritte auf:
a. Erfassen von Patientendaten eines Patienten in einem Rechner mit Patientenverwaltungssystem,
b. Platzieren des Patienten,
c. Einrichten einer mit dem Rechner verbundenen Erweiterte Realität-Vorrichtung,
d. Vorbereiten eines mit dem Rechner und der Erweiterte Realität-Vorrichtung verbundenen Robotersystems aufweisend einen Roboterarm mit einem Ultraschallkopf und Platzieren auf dem Patienten,
e. Festlegen eines Ultraschallbereiches für eine periphere endovaskuläre Intervention auf dem Patienten,
f. Automatisches Durchführen eines Ultraschalls über den Roboterarm in dem Ultraschallbereich,
g. Auswerten der Ultraschallergebnisse und Visualisierung über die Erweiterte Realität-Vorrichtung,
h. Automatische Rückkehr des Roboterarmes an den Beginn des Ultraschallbereiches,
i. Durchführen der peripheren endovaskulären Intervention umfassend: Manuelles Einbringen von Interventionsmaterial in den Patienten mithilfe der Daten der Erweiterten Realität-Vorrichtung, Erkennen des Interventionsmaterials durch den Roboterarm und automatisches Durchführen eines das Einbringen begleitenden Ultraschalls;
j. Automatisches Durchführen eines Ultraschalls über den Roboterarm in dem Ultraschallbereich nach Ende des Interventionsvorganges und
k. Postinterventionelles Verarbeiten der bei der Intervention gewonnenen Ultraschalldaten.

Das Platzieren des Roboterarmes mit Ultraschallkopf auf dem Patienten kann entweder manuell oder auch automatisch durchgeführt werden. Bevor der Roboterarm mit Ultraschallkopf auf dem Patienten platziert wird, wird der Roboterarm manuell auf der gegenüberliegenden Seite des zu behandelnden Organsystems des Patienten platziert. Anschließend wird ein Systemcheck zur Überprüfung der korrekten Funktionsweise des Robotersystems durchgeführt. Ist die Funktionsweise des Roboterarms fehlerlos, so wird der Ultraschallkopf an den Roboterarm angeschlossen. Wird das abschließende Platzieren des Roboterarmes mit Ultraschallkopf auf dem Patienten manuell durchgeführt, so wird der erste Wegpunkt, also die Ausgangsposition, sprachgesteuert oder virtuell manuell festgelegt. Alternativ kann der Roboterarm mit Ultraschallkopf mit Hilfe eines optischen Patientenerkennungssystems automatisch platziert werden. Das Zielorgan wird über den Roboterarm aufgesucht und von diesem automatisch als solches erkannt.

Das Festlegen des Ultraschallbereiches auf dem Patienten kann manuell oder automatisch durchgeführt werden. Wird der Ultraschallbereich manuell festgelegt, so werden Start- und Endpunkt durch manuelles Führen des Roboterarmes an entsprechende Positionen definiert. Die automatische Festlegung des Ultraschallbereichs wird anhand der zuvor in das System eingelesenen Patientendaten aus dem Patientenverwaltungssystem vorgenommen.

Das Festlegen eines Ultraschallbereichs auf einem Patienten beinhaltet das Festlegen von Intervallen in ebendiesem Ultraschallbereich zur Messung der endovaskulären Strömungsgeschwindigkeit. Die endovaskuläre Strömungsgeschwindigkeit wird für die Berechnung einer virtuellen-Ultraschall-Angiographie und zur Generierung eines virtuellen dreidimensionalen Gefäßmodells genutzt.

Wenn die Durchführung des Ultraschalls in zwei Ebenen durchgeführt wird, so werden detailliertere Erkenntnisse über das zu untersuchende Organsystem / Gefäßsystem erhalten als bei Durchführung eines Ultraschalls in einer Ebene. Dies resultiert in einer realitätsgetreueren Darstellung als Folge der Auswertung der gewonnenen Daten.

Dadurch, dass das Auswerten des Ultraschalls zur Erstellung einer virtuellen-Ultraschall-Angiographie und eines virtuellen dreidimensionalen Gefäßmodells in der Erweiterte Realität-Vorrichtung dient, wird es für den Operateur möglich das Innere des Patienten virtuell über die erstellten Modelle in dem definierten Bereich über die Erweiterte Realität-Vorrichtung zu betrachten. Die virtuelle-Ultraschall-Angiographie dient der Evaluation der Gefäßpathologie, beispielsweise dem Auffinden hämodynamisch relevanter Stenosen, sowie als Vergleichsbasis für die Abschlussangiographie. Bei Visualisierung der virtuellen-Ultraschall-Angiographie über die Erweiterte Realität-Vorrichtung ist es möglich die Flusskinetik in einer dynamischen Abfolge, wobei die Anzeigedauer gewählt werden kann, ähnlich einer konventionellen Angiographie anzeigen zu lassen. Die Flussgeschwindigkeiten werden dabei wie in der Duplexsonographie farbkodiert dargestellt. Des Weiteren kann sich der Operateur einzelne lokale Flussgeschwindigkeiten durch virtuellen Fingerzeig auf die virtuelle-Ultraschall-Angiographie anzeigen lassen. Die lokalen Flussgeschwindigkeiten können dabei mit einem Wegpunkt verbunden werden, um später wieder virtuell manuell oder per Sprachsteuerung abgerufen werden zu können.

Da die Wegpunkte für die virtuelle-Ultraschall-Abschluss-Angiographie automatisch übernommen werden, können diese dann postinterventionell mit den präinterventionellen Daten verglichen werden. So ist es möglich den Interventionserfolg zu quantifizieren.

Das virtuelle dreidimensionale Gefäßmodell wird während der manuellen Intervention als Navigationshilfe **über** die Erweiterte Realität-Vorrichtung ausgegeben. Anhand des dreidimensionalen Gefäßmodells wird dem Operateur während der Intervention über die Erweiterte Realität-Vorrichtung in Echtzeit die Katheterposition / die Position des Ultraschallkopfes angezeigt.

Wenn das Einbringen von Interventionsmaterial in den Patienten von proximal nach distal durchgeführt wird, so wird die standardmäßig genutzte Richtung für das Einbringen von Interventionsmaterial genutzt. Vor Beginn der Intervention kehrt der Roboterarm mit Ultraschallkopf automatisiert an den ersten Wegpunkt / Ausgangspunkt zurück. Es folgt der Beginn der Intervention mit einer sonographisch gesteuerten manuellen Punktion des Gefäßes. Eine Schleuse wird manuell eingebracht und der Interventionsdraht vorgeschoben. Der Interventionsdraht wird vom System erkannt und die Spitze des Drahtes automatisiert beim Vorschieben nach distal verfolgt.

Dadurch, dass über die Erweiterte Realität-Vorrichtung die Ausgabe mehrerer verschiedener Datensätze parallel erfolgen kann, ist es hierbei möglich, dass sowohl das Ultraschallbild, also die Drahtverfolgung, als auch eine Navigationsübersicht, also die Position des Ultraschallkopfes relativ zum virtuellen dreidimensionalen Gefäßmodell, zeitgleich anzeigen zu lassen. Dies vereinfacht dem Operateur die Durchführung der Intervention.

Da in der virtuellen-Ultraschall-Angiographie und / oder im virtuellen dreidimensionalen Gefäßmodell präinterventionelle und / oder interventionelle Wegpunkte gesetzt werden können, ist es möglich präinterventionelle und postinterventionelle Ultraschallergebnisse miteinander zu vergleichen.

Wenn präinterventionelle und postinterventionelle Ultraschallergebnisse miteinander verglichen werden, so kann der Interventionserfolg quantifiziert werden. Beim Vergleich der virtuellen-Ultraschall-Angiographie mit der virtuellen-Ultraschall-Abschluss-Angiographie kann beispielsweise eine Steigerung der Gefäßperfusion durch die Intervention dargestellt werden.

Während der Intervention können genau wie vor der Intervention an jeder beliebigen Stelle Wegpunkte gesetzt werden. Auf der virtuellen dreidimensionalen Darstellung des Gefäßes werden die Wegpunkte visuell dargestellt und markiert. Bei Bedarf kann der Roboterarm mit Ultraschallkopf automatisch sprachgesteuert oder virtuell manuell durch Berühren des Wegpunktes an jeden gesetzten Wegpunkt zurückkehren. Diese Funktion kann auch zum Ausmessen beispielsweise der korrekten Stentgröße verwendet werden über Messen der Länge zwischen zwei Wegpunkten und Berechnung des mittleren Gefäßdurchmessers zwischen ebendiesen Wegpunkten. Das Ergebnis wird über die Erweiterte Realität-Vorrichtung angezeigt.

Während der Intervention können durchgängig Echtzeitdaten abgerufen, beziehungsweise eingeblendet werden. Bei der Inflation eines Angioballons können beispielsweise die Mannometerangaben virtuell über die Erweiterte Realität-Vorrichtung eingeblendet werden. Dem Operateur liegen so Echtzeitbild und Ballondruck zusammen vor und er muss nicht ständig die Blickrichtung ändern.

Wenn der Rechner einen Zugriff auf das Materiallogistiksystem aufweist, so wird ein Zugriff auf ebendieses für einen Operateur auch während einer Intervention möglich. Hat der Operateur während einer Intervention beispielsweise eine benötigte Stentgröße ermittelt, so wird über das Materiallogistiksystem der Zugriff auf die Systemdatenbank möglich. In der Systemdatenbank sind beispielsweise alle vorrätigen Stentgrößen hinterlegt. Das System kann an dieser Stelle einen Materialvorschlag machen. Wird das Material dann verwendet, so wird es automatisch im System als verbraucht markiert. Diese Funktion kann äquivalent für verschiedenste Interventionsmaterialien wie beispielsweise auch Ballongrößen für Angioplastien genutzt werden. Der Zugriff auf das Materiallogistiksystem kann entweder über Sprachsteuerung oder virtuell manuell erfolgen.

Wenn während der Durchführung des Navigationsverfahrens das Abrufen von Patientendaten notwendig wird, so kann dieses über Sprachsteuerung oder auch virtuell manuell erfolgen. Der Zugriff auf das Patientenverwaltungssystem ist über den Rechner des Navigationssystems gegeben.

Nachfolgend wird ein Ausführungsbeispiel der erfindungsgemässen Vorrichtung am Beispiel einer peripheren endovaskulären Intervention der Arteria femoralis superficialis (AFS) beschrieben.

Es zeigen:
- Abb. 1: eine schematische Darstellung der Erkennung der Arteria femoralis superficialis mit anschließendem automatisierten Ultraschallscan durch einen Roboterarm;
- Abb. 2: eine schematische Darstellung des automatisierten Ultraschallscans durch den Roboterarm aus Abbildung 1 in axialer Ansicht;
- Abb. 3: eine schematische Darstellung der Erkennung eines Interventionsdrahtes im Gefäßsystem bei Einführen ebendieses Drahtes;
- Abb. 4: eine schematische Darstellung der automatischen Nachführung des Roboterarms bis zur Zielläsion und automatisierte Ultraschallverfolgung;
- Abb. 5: eine schematische Darstellung der automatischen Steuerung des Ultraschallkopfes auf den Interventionsfokus und
- Abb. 6: eine schematische Darstellung der duplexsonographischen Kontrolle nach der Intervention.

An dieser Stelle soll darauf hingewiesen werden, dass funktionsgleiche Elemente mit einheitlichen Bezugszeichen versehen sind.

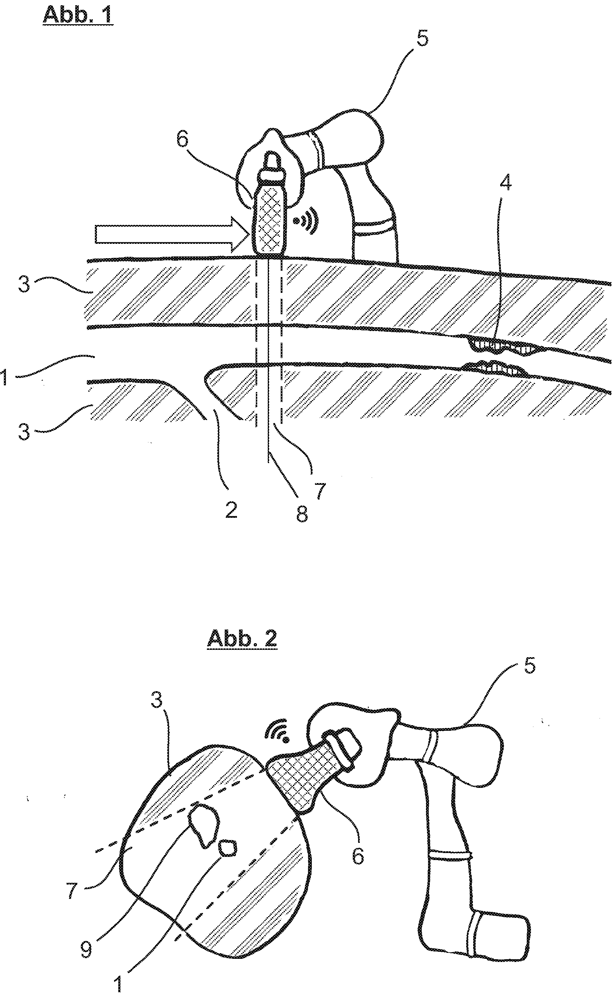

In **Abb. 1** ist eine schematische Darstellung der Erkennung der Arteria femoralis superficialis 1 dargestellt. Der Abgang der Aorta profunda femoris 2 von der Aorta femoralis superficialis 1 ist ersichtlich. Ein Roboterarm 5 mit einem Ultraschallkopf 6 berührt die Patientenoberfläche und fährt von einem Startpunkt aus an dieser entlang. Der Ultraschallkopf 5 hat einen definierten Ultraschallsektor 7 und führt durch das Gewebe 3 des Patienten hindurch einen Ultraschall zur Diagnostik/Evaluation eines Abschnittes der Arteria femoralis superficialis 1 durch. Vom Operateur wurde ein Wegpunkt 8 gesetzt. In einem Bereich der Aorta femoralis sperficialis 1 ist eine Stenose 4 dargestellt. Diese Stenose 4 wird vom Roboterarm 5 mit Ultraschallkopf 6 während des automatisierten Ultraschallscans evaluiert.

Während der Durchführung des automatisierten Ultraschallscans können vom Operateur eine beliebige Anzahl an Wegpunkten 8 gesetzt werden. Der Startpunkt und der Endpunkt für den automatisierten Ultraschallscan wurden entweder anhand der bereits vorhandenen Patientendaten festgelegt oder manuell durch Führen des Roboterarms an die entsprechenden Positionen definiert. Der automatisierte Ultraschallscan wird von proximal nach distal in zwei Ebenen durchgeführt. Während des automatisierten Ultraschallscans werden in zuvor festgelegten Intervallen Messungen der Strömungsgeschwindigkeit für spätere Berechnungen durchgeführt.

**Abb. 2** zeigt eine schematische Darstellung des automatischen Ultraschallscans aus Abbildung 1 in axialer Ansicht. In dem Gewebe 3 des Patienten sind der Femur 9 und die Aorta femoralis superficialis 1 dargestellt. Sowohl der Femur 9 als auch die Aorta femoralis superficialis 1 befinden sich im Ultraschallsektor 7 des Roboterarmes 5 mit Ultraschallkopf 6.

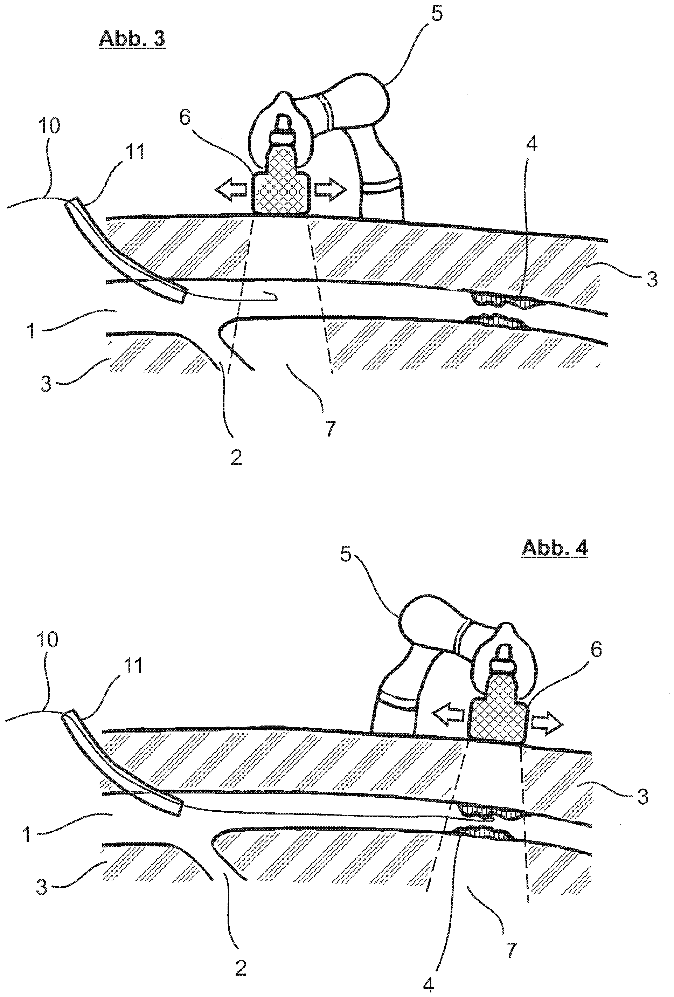

In **Abb. 3** ist eine schematische Darstellung der Erkennung des Interventionsdrahtes10 im Gefäßsystem bei Einführen des Interventionsdrahtes 10 gezeigt. Der Interventionsdraht 10 wird hier durch eine Schleuse 11 in die Aorta femoralis superficialis 1 eingeführt.

Die Spitze des Interventionsdrahtes 10 wird vom System erkannt und vom Roboterarm 5 mit Ultraschallkopf 6 automatisiert verfolgt. Die Spitze des Interventionsdrahtes 10 befindet sich somit kontinuierlich im Ultraschallsektor 7 des Roboterarmes 5 mit Ultraschallkopf 6.

**Abb. 4** zeigt eine schematische Darstellung der automatischen Nachführung des Roboterarmes 5 aus den vorherigen Abbildungen bis zur Zielläsion, also der Stenose 4.

Dem Operateur werden während des Vorganges in der Erweiterte Realität-Vorrichtung sowohl das Ultraschallbild für die Drahtverfolgung, als auch eine Navigationsübersicht für eine Darstellung des Ultraschallkopfes 6 relativ zu dem virtuellen dreidimensionalen Gefäßmodell angezeigt.

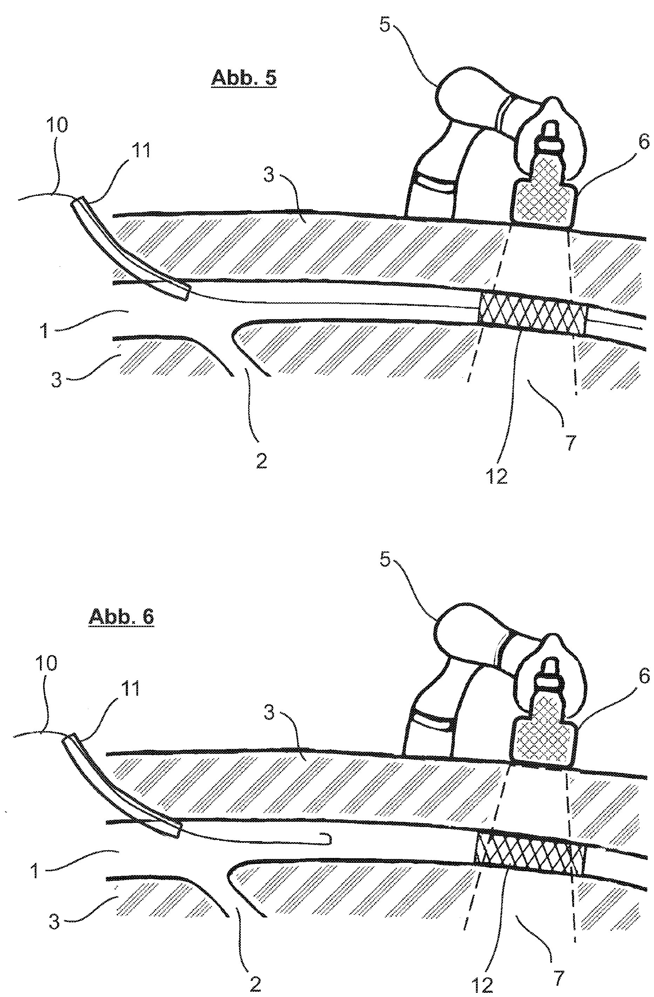

In **Abb. 5** ist eine schematische Darstellung der automatischen Steuerung des Ultraschallkopfes 6 auf den Interventionsfokus gezeigt. Im Bereich der Stenose 4 aus den vorherigen Abbildungen ist ein Stent 12 eingebracht worden. Der Ultraschallsektor 7 des Roboterarmes 5 mit Ultraschallkopf 6 befindet sich in ebendiesem Bereich von Interesse.

Das Einbringen des Stents 12 ist mithilfe des Interventionsdrahtes 10 erfolgt.

**Abb. 6** zeigt eine schematische Darstellung der duplexsonographischen Kontrolle des Bereiches der Aorta femoralis superficialis 1, in welchen der Stent 12 eingebracht wurde, also nach der Intervention. Der Ultraschallsektor 7 des Roboterarmes 5 mit Ultraschallkopf 6 umfasst den neu eingebrachten Stent 4. Der Interventionsdraht 10 wird in dieser Abbildung über die Schleuse 11 hinaus aus dem Patienten zurückgeführt.

Während der duplexsonographischen Kontrolle werden erneut Messungen der Strömungsgeschwindigkeiten in den zuvor festgelegten Intervallen durchgeführt.

Es wird eine virtuelle Ultraschall-Abschluss-Angiographie erstellt und diese mit der Ausgangsangiographie verglichen. Hiermit kann der Interventionserfolg quantifiziert werden.

Die erfindungsgemässe Vorrichtung erlaubt die Durchführung von peripheren, endovaskulären Interventionen, die komplett röntgenstrahlungsfrei und kontrastmittelfrei sind. Wie zuvor beschrieben führt dies zu einer deutlichen Minimierung gesundheitlicher Risiken für den Operateur und den Patienten bei der Durchführung ebendieser.

### Bezugszeichenliste

- 1: Aorta femoralis superficialis
- 2: Aorta profunda femoris
- 3: Gewebe
- 4: Stenose
- 5: Roboterarm
- 6: Ultraschallkopf
- 7: Ultraschallsektor
- 8: Wegpunkt
- 9: Femur
- 10: Interventionsdraht
- 11: Schleuse
- 12: Stent

## Patentansprüche

1. Ultraschall-Erweiterte Realität-Peripher Endovaskulär Intervention-Navigationsanordnung, zur Anwendung an einem Patienten, an welchem eine periphere endovaskuläre Intervention durchgeführt werden soll, umfassend
- eine Erweiterte Realität-Vorrichtung;
- ein Robotersystem mit einem Roboterarm (5) mit Ultraschallkopf (6);
- einen Rechner mit Zugriff auf ein Patientenverwaltungssystem, in welchem der Patient hinterlegt ist;
wobei
- die Erweiterte Realität-Vorrichtung als Erweiterte Realität-Dreidimensional-Brille ausgebildet ist,
- der Patient ein Lebewesen oder ein Modell zu Simulationszwecken ist;
- Rechner, Erweiterte Realität-Vorrichtung und Robotersystem über eine Software miteinander verbunden sind und kommunizieren;
- der Roboterarm (5) und die Erweiterte Realität-Vorrichtung sprachgesteuert und / oder virtuell manuell gesteuert ausgebildet sind; wobei
- der Roboterarm (5) mit Ultraschallkopf (6) zur Durchführung eines automatisierten Ultraschalls auf einem Patienten ausgebildet ist,
- die Erweiterte-Realität-Vorrichtung für eine visuelle Darstellung der Ergebnisse des Ultraschalls ausgebildet ist,
- bei Durchführung der Intervention in der Erweiterte Realität-Vorrichtung
- verschiedene Ultraschallergebnisse visuell zeitgleich parallel und / oder kombiniert abbildbar und / oder aufrufbar sind,
- zeitaktuelle Ultraschallergebnisse durch den Roboterarm (5) mit Ultraschallkopf (6) übermittelt werden und visualisierbar sind und
- Echtzeitdaten abrufbar und / oder einblendbar sind,
wobei das Navigationssystem dazu konfiguriert ist ein Navigationsverfahren durchzuführen welches die folgenden Schritte umfasst
a. Erfassen von Patientendaten des Patienten in dem Rechner mit dem Patientenverwaltungssystem;
b. Einrichten der mit dem Rechner verbundenen Erweiterte Realität-Vorrichtung;
c. Vorbereiten des mit dem Rechner und der Erweiterte Realität-Vorrichtung verbundenen Robotersystems und Platzieren auf dem platzierten Patienten;
d. Festlegen eines Ultraschallbereiches für eine periphere endovaskuläre Intervention auf dem Patienten, welches das Festlegen von Intervallen in ebendiesem Ultraschallbereich zur Messung der endovaskulären Strömungsgeschwindigkeit beinhaltet;
e. Automatisches Durchführen eines Ultraschalls über den Roboterarm (5) in dem Ultraschallbereich;
f. Auswerten der Ultraschallergebnisse zur Erstellung einer virtuellen-Ultraschall-Angiographie und eines virtuellen dreidimensionalen Gefäßmodells sowie Visualisierung über die Erweiterte Realität-Vorrichtung, wobei das virtuelle dreidimensionale Gefäßmodell während einer manuellen Intervention als Navigationshilfe dient;
g. Automatische Rückkehr des Roboterarmes (5) an den Beginn des Ultraschallbereiches;
h. Durchführen der peripheren endovaskulären Intervention umfassend:
- Erkennen von Interventionsmaterial, das mithilfe der Daten der Erweiterten Realität-Vorrichtung manuell in den Patienten eingebracht wird, durch den Roboterarm, und
- Automatisches Durchführen eines das Einbringen begleitenden Ultraschalls, wobei die Position des Ultraschallkopfes (6) während der manuellen Intervention über die Erweiterte Realität-Vorrichtung ausgegeben wird;
i. Automatisches Durchführen eines Ultraschalls über den Roboterarm (5) in dem Ultraschallbereich nach Ende des Interventionsvorganges und
j. Postinterventionelles Verarbeiten der bei der Intervention gewonnenen Ultraschalldaten.

2. Navigationsanordnung nach Anspruch 1,
**dadurch gekennzeichnet, dass**
der Rechner einen Zugriff auf das Materiallogistiksystem aufweist, wobei über die Systemdatenbank des Materiallogistiksystems anhand der Ultraschallergebnisse Materialvorschläge für das Interventionsmaterial ausgegeben werden und verbrauchtes Material markiert wird.

## Claims

1. Ultrasound-based augmented reality peripheral endovascular intervention navigation system for use on a patient on whom a peripheral endovascular intervention is to be performed, comprising:
- an augmented reality device;
- a robot system with a robot arm (5) with an ultrasound probe (6);
- a computer with access to a patient management system in which the patient is stored; wherein
- the augmented reality device is in the form of augmented reality three-dimensional glasses,
- the patient is a living being or a model for simulation purposes;
- the computer, augmented reality device and robot system are connected to one another and communicate via software;
- the robot arm (5) and the augmented reality device are voice-controlled and/or virtually manually controlled;
wherein
- the robot arm (5) is designed with an ultrasound head (6) for performing an automated ultrasound on a patient,
- the augmented reality device is designed for a visual representation of the results of the ultrasound,
- during the intervention in the augmented reality device
- various ultrasound results can be visually displayed and/or called up simultaneously in parallel and/or in combination,
- real-time ultrasound results are transmitted by the robot arm (5) with the ultrasound head (6) and can be visualised, and
- real-time data can be retrieved and/or displayed,
wherein the navigation system is configured to carry out a navigation procedure comprising the following steps:
a. recording patient data in the computer with the patient administration system;
b. setting up the augmented reality device connected to the computer;
c. preparing the robot system connected to the computer and the augmented reality device and placing it on the patient;
d. defining an ultrasound area for a peripheral endovascular intervention on the patient, which involves defining intervals in this ultrasound area for measuring endovascular flow velocity;
e. automatically performing an ultrasound in the ultrasound range via the robot arm (5);
f. evaluating the ultrasound results to create a virtual ultrasound angiography and a virtual three-dimensional vascular model and visualising them via the augmented reality device, with the virtual three-dimensional vascular model serving as a navigation aid during a manual intervention;
g. Automatic return of the robot arm (5) to the start of the ultrasound range;
h. Performing the peripheral endovascular intervention, comprising:
- Recognising, by the robotic arm, interventional material that is manually inserted into the patient using the augmented reality device data; and
- automatically performing an ultrasound accompanying the insertion, whereby the position of the ultrasound probe(6) during the manual intervention is output via the augmented reality device;
i. Automatically performing an ultrasound via the robotic arm (5) in the ultrasound area after the end of the intervention procedure; and
j. Post-interventional processing of the ultrasound data obtained during the intervention.

2. Navigation system according to claim 1,
**characterised in that**
the computer has access to the material logistics system, whereby material suggestions for the intervention material are issued via the system database of the material logistics system on the basis of the ultrasound results and used material is marked.

## Revendications

1. Dispositif de navigation d'intervention endovasculaire périphérique en réalité augmentée par ultrasons, destiné à être utilisé sur un patient sur lequel une intervention endovasculaire périphérique doit être effectuée, comprenant:
- un dispositif de réalité augmentée;
- un système robotique comprenant un bras robotique (5) avec une tête à ultrasons (6);
- un ordinateur ayant accès à un système de gestion de patients dans lequel le patient est enregistré;
dans lequel
- le dispositif de réalité augmentée est conçu comme des lunettes de réalité augmentée tridimensionnelles,
- le patient est un être vivant ou un modèle à des fins de simulation;
- l'ordinateur, le dispositif de réalité augmentée et le système robotique sont reliés et communiquent entre eux par le biais d'un logiciel;
- le bras robotisé (5) et le dispositif de réalité augmentée sont à commande vocale et/ou à commande manuelle virtuelle;
dans lequel
- le bras robotisé (5) est conçu avec une tête à ultrasons (6) pour effectuer une échographie automatisée sur un patient,
- le dispositif de réalité augmentée est conçu pour une représentation visuelle des résultats de l'ultrason,
- lors de l'exécution de l'intervention dans le dispositif de réalité augmentée
- différents résultats d'ultrasons peuvent être représentés et/ou appelés visuellement simultanément en parallèle et/ou de manière combinée,
- les résultats actuels des ultrasons sont transmis par le bras robotisé (5) avec la tête à ultrasons (6) et peuvent être visualisés, et
- des données en temps réel peuvent être appelées et / ou affichées,
dans lequel le système de navigation est configuré pour exécuter un procédé de navigation qui comprend les opérations suivantes:
a. Saisie des données du patient dans l'ordinateur avec le système de gestion des patients;
b. Configuration du dispositif de réalité augmentée connecté à l'ordinateur;
c. Préparer le système robotique connecté à l'ordinateur et au dispositif de réalité augmentée et le placer sur le patient placé;
d. Définition d'une zone d'ultrasons pour une intervention endovasculaire périphérique sur le patient, ce qui implique la définition d'intervalles dans cette zone d'ultrasons pour mesurer le débit endovasculaire;
e. Exécution automatique d'une échographie par le bras robotisé (5) dans la zone d'échographie
f. Analyser les résultats des ultrasons pour créer une angiographie virtuelle par ultrasons et un modèle vasculaire tridimensionnel virtuel et les visualiser par l'intermédiaire du dispositif de réalité augmentée, le modèle vasculaire tridimensionnel virtuel servant d'aide à la navigation pendant une intervention manuelle;
g. Retour automatique du bras robotisé (5) au début de la zone d'échographie;
h. Réalisation de l'intervention endovasculaire périphérique comprenant:
- la reconnaissance par le bras robotique du matériel d'intervention qui est introduit manuellement dans le patient à l'aide des données du dispositif de réalité augmentée, et
- Réalisation automatique d'une échographie accompagnant l'insertion, la position de la tête à ultrasons(6) étant fournie par le dispositif de réalité augmentée pendant l'intervention manuelle;
i. Exécution automatique d'une échographie par le bras robotisé (5) dans la zone d'échographie après la fin de l'intervention ; et
j. traitement post-interventionnel des données ultrasonores obtenues lors de l'intervention.

2. Système de navigation selon la revendication 1,
**caractérisé en ce que**
l'ordinateur présente un accès au système logistique de matériel, des propositions de matériel pour le matériel d'intervention étant émises par l'intermédiaire de la base de données du système logistique de matériel à l'aide des résultats des ultrasons et le matériel usagé étant marqué.
